(19) Europäisches Patentamt
European Patent Office
Office européen des brevets

(11) Publication number: **0 101 554**
**B1**

(12) # EUROPEAN PATENT SPECIFICATION

(45) Date of publication of patent specification: **15.10.86**

(51) Int. Cl.[4]: **C 07 J 9/00,** A 61 K 31/575

(21) Application number: **83106708.7**

(22) Date of filing: **08.07.83**

(54) **New derivatives of biliary acids, process for the production thereof and pharmaceutical compositions containing the same.**

(30) Priority: **29.07.82 IT 2263282**
**01.07.83 IT 2190983**

(43) Date of publication of application:
**29.02.84 Bulletin 84/09**

(45) Publication of the grant of the patent:
**15.10.86 Bulletin 86/42**

(84) Designated Contracting States:
**AT BE CH DE FR GB IT LI LU NL SE**

(56) References cited:

**TETRAHEDRON LETTERS, no. 9, February 1979, pages 763-766, Pergamon Press Ltd., Oxford (GB); MASAJI ISHIGURO et al.: "Stereoselective synthesis of demethylgorgosterol".**

(73) Proprietor: **LEHNER A.G.**
**Birsfelderstrasse 44**
**CH-4132 Muttenz (CH)**

(72) Inventor: **Castagnola, Virginio**
**Via Crimea 23**
**I-20100 Milan (IT)**
Inventor: **Frigerio, E.Giuliano**
**Via Vittorio Veneto 107**
**Bresso-Milan (IT)**
Inventor: **Pellicciari, Roberto**
**Via Ulisse Rocchi 60**
**I-06100 Perugia (IT)**

(74) Representative: **Braun, André et al**
**A. Braun, Braun, Héritier, Eschmann AG**
**Patentanwälte Holbeinstrasse 36-38**
**CH-4051 Basel (CH)**

**EP 0 101 554 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a series of biliary acids derivatives having the formula (I):

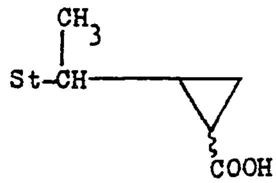

(I)

wherein St is a 17-etiocholanyl radical, having two or more hydroxyl groups in either the α and β configuration, some of which being optionally replaced by keto groups, and their pharmaceutically acceptable salts with alkaline or alkali-earth metals, or with organic bases.

The compounds of formula I, and their salts above cited, are endowed with interesting pharmacological properties. Namely, they exhibit a higher hydrophobicity in comparison with ursodeoxycholic acid, a widely used choleretic and stone dissolving agent. As a consequence compounds I are less metabolized by the liver and exhibit higher and more favourable pharmacological activities and a longer duration of action. The invention relates therefore also to pharmaceutical compositions containing as the active principle one or more compounds of formula (I) or their pharmaceutically acceptable salts.

Non-limiting examples of compounds I of the present invention are the following ones:

-3α,7α,12α-trihydroxy-5β-cholan-22,23-methylene-24-oic acid (22,23-methylene-cholic acid) (Ia).

-3α,7β,12α-trihydroxy-5β-cholan-22,23-methylene-24-oic acid (Ib).

-3α,7β-dihydroxy-5β-cholan-22,23-methylene-24-oic acid (22,23-methanate-ursodeoxycholic acid) (Ic).

-3α,7α-dihydroxy-5β-cholan-22,23-methylene-24-oic acid (22,23-methanate-chenodeoxycholic acid) (Id).

-3α-hydroxy-7-keto-5β-cholan-22,23-methylene-24-oic acid (3α-hydroxy-7-keto-22,23-methylene-cholanic acid) (Ie).

The invention relates also to a process for the preparation of the compounds of formula (I), characterized by hydrolyzing corresponding esters having formula (II)

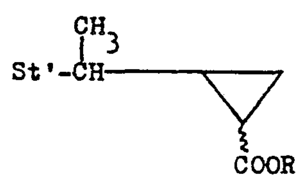

(II)

wherein St' represents a 17-etiocholanyl radical having two or more acyloxy groups in either α and β configuration, or keto groups, and R is a $C_1$—$C_4$ alkyl radical. Preferably, the acyloxy groups are acetoxy groups and R is a methyl or ethyl radical.

The hydrolysis is preferably carried out in a basic medium, for instance in an aqueous alcoholic solution of an alkali metal hydroxide, such as sodium or potassium hydroxide, at temperatures ranging from 20 to 100°C, preferably at the reflux temperature of the solvent mixtures, for between 1 and 12 hours.

Compounds II are in turn obtained by reacting 24-nor-cholenes (III)

$CH_3$
|
St'—CH—CH=$CH_2$ (III)

wherein St' has the above mentioned meaning, with alkyl diazoacetates $N_2CH$—COOR (wherein R has the above specified meaning) in presence of Cu-Bronze in inert solvents, for instance cyclohexane.

Finally, the 24-norcholenes (III) are prepared by treating the acids (IV)

$CH_3$
|
St'—CH—$CH_2$—$CH_2$—COOH (IV)

wherein St' has the above specified meanings, with lead tetraacetate (in presence of $Cu^{++}$ ions, preferably of cupric acetate) in pyridine.

The process according to the invention will be more easily understood from the scheme hereinbelow reported for the preparation of 22,23-methylene-ursodeoxycholic acid (Ic):

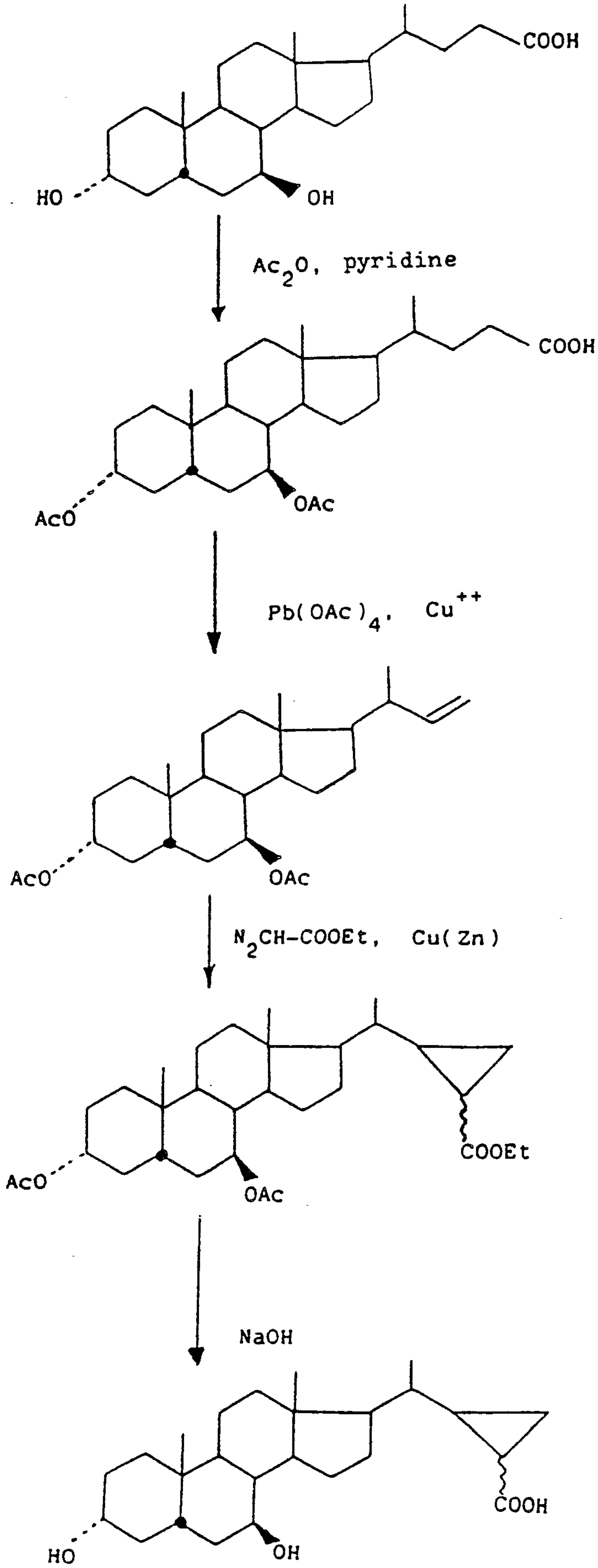


(V)

(VI)

(Ic)

It is plain that the esters (II) of the present invention can be present in two isomeric forms from the steric point of view and that this influences of course the acids (I) obtained from the esters themselves. Of course, the invention refers to all possible stereoisomers of the claimed compounds.

The following examples illustrate the process according to the invention, without limiting in any way the scope thereof.

Example 1

a) 3α,7β-Diacetoxy-$\Delta^{22}$-24-norcholene (V)

A mixture of ursodeoxycholic acid (I) (40 g), acetic anhydride (60 ml), and pyridine (48 ml) is left to react overnight. The reaction mixture is poured onto ice-water and extracted with chloroform. The organic phase is washed with 10% HCl until acid, then with water until neutral, dried over $MgSO_4$ and evaporated. The crude product so obtained, without further purification, $Pb(OAc)_4$ (70 g), $Cu(OAc)_2$ (4 g), and pyridine (10 ml), are dissolved in anhydrous benzene (2 l), and refluxed under stirring in a nitrogen atmosphere for 4 hours. After cooling, the reaction mixture is treated with ethylene glycol (2 × 200 ml), diluted with ether (1 l), washed with 10% KOH (3 × 150 ml), washed with water until neutral dried over $Na_2SO_4$ evaporated.

After column chromatography ($SiO_2$; h = 30 cm, ϕ = 4.3 cm; petroleum ether/ethylether 96/4) 8.5 g of V (m.p. 112—114°C) were obtained.

b) Ethyl ester of 3α,7β-diacetoxy-22,23-methylene-ursodeoxycholic acid (VI)

A solution of ethyl diazoacetate (EDA) (3.1 ml, 0.03 mol) in cyclohexane (80 ml), was dipped very slowly (2 days) into a suspension of V (8.5 g, 0.02 mol) and Cu-Bronze (≃ 10 g) in anhydrous cyclohexane (170 ml), and then refluxed under stirring in a nitrogen atmosphere.

The reaction mixture after cooling was filtered and evaporated.

The residue was subjected to column chromatography ($SiO_2$; h = 35 cm; ϕ = 3 cm).

By eluting with petroleum ether containing 2% of ethyl ether, and increasing the percent of ethyl ether, and increasing the percent of ethyl ether by 1% every 200 ml of eluent until reaching 7%, there were obtained: a fraction containing V (4.7 g), an α fraction (0.55 g), an α + β fraction (0.9 g), and a β fraction (1.8 g). The NMR spectrum of the α and β fractions is in agreement with the structyure VI.

c) 22,23-Methylene-ursodeoxycholic acid (Ic)

A solution of the β chromatographic fraction (1.8 g) in 15% NaOH in $EtOH/H_2O$ (6:4, 180 ml) was refluxed under stirring for 4 h. After cooling, the reaction mixture was diluted with $H_2O$, acidified with conc. HCl, and extracted with chloroform. The organic phase was dried over $Na_2SO_4$ and evaporated. By column chromatography ($SiO_2$; h = 35 cm; ϕ = 2.1 cm) and eluting with chloroform a substance C (240 mg) (m.p. 133—136°C) was obtained; eluting with chloroform with 2% of methanol a substance D (270 mg) (m.p. 153—59°C) in addition to the mixture of the two C + D (370 mg) was obtained. The chemco-physical properties of C and D are in agreement with the structure Ic. Similarly, by hydrolysis of the α fraction two substances A (m.p. 242—244°C) and B (m.p. 252—255°C) are obtained; the structure Ic can be proposed also for them.

Example 2

a) 3α-Acetoxy-7-keto-$\Delta^{22}$-24-norcholene

A mixture of 3α-hydroxy-7-keto-5β-cholan-24-oic acid (nutriacholic acid) (40 g), acetic anhydride (60 ml), and pyridine (58 ml), is left to react overnight, thereafter it is poured onto ice-water and extracted with chloroform.

The organic phase was washed with 10% HCl until acid, then with water until neutral, dried over $MgSO_4$ and evaporated. The crude reaction product so obtained, without further purification, $Pb(OAc)_4$ (70 g), $Cu(OAc)_2$ (4 g) and pyridine (10 ml), are dissolved in anhydrous benzene (2 l), and refluxed under stirring in a nitrogen atmosphere for 4 hours. After the cooling the reaction mixture is treated with ethylene glycol (2 × 200 ml), diluted with ether (1 liter), washed with 10% KOH (3 × 150 ml), washed with water until neutral, dried over $Na_2SO_4$ and evaporated. After column chromatography ($SiO_2$; h = 30 cm; ϕ = 3 cm; petroleum ether) 8.5 g of 3α-acetoxy-7-keto-$\Delta^{22}$-24-norcholene are obtained.

b) Ethyl ester of 3α-acetoxy-7-keto-22,23-methylenecholanic acid

A solution of ethyl diazoacetate (EDA) (3.5 ml, 34 mmoles) in cyclohexane (80 ml) was dripped very slowly (about 2 days) into a suspension of the compound obtained in a) (8.5 g; 22 mmol) and Cu-Bronze (≃ 10 g) in anhydrous cyclohexane (200 ml), and then refluxed in a nitrogen atmosphere.

After cooling the reaction mixture was filtered and evaporated. The thin layer chromatography shows the formation of products having a lower $R_f$ than the starting substrate, giving evidence of the formation of the desired product.

Example 3

a) 3α,7β-Diacetoxy-24-nor-5β-chol-22-ene

A mixture of 3α,7α-diacetoxy-5β-cholanoic acid (16.9 g), lead tetraacetate (33.46 g), copper acetate (1.7 g) and anhydrous pyridine (2 ml) in anhydrous benzene (1 l) is stirred under reflux for 4 hours. The solids are collected on a Celite® filter. After evaporation of the benzene, the residue is dissolved in ether, washed with 5% aqueous HCl and a 10% sodium bicarbonate solution. The crude extract is chromatographed on silica gel column (750 g). By elution with ethyl acetate-hexane 3:7, the 3α,7α-

diacetoxy-24-nor-5β-chol-22-ene is obtained (8.3 g, 82%), m.p. 134°C, I.R.: 1730, 1640 $cm^{-1}$. MS: $M^+ = 430$, m/e = 370, 255, 256.

b) Ethyl 3α,7α-diacetoxy-22,23-methylene-5β-cholanate

A solution of ethyl diazoacetate (3.2 g, 28.1 mmol) in anhydrous methylene chloride (90 ml) was added dropwise in about 14 h to a suspension of dirhodium (II) tetraacetate (75 mg, 0.17 mmol) and of the compound obtained in a), (4 g, 9.3 mmol) in anhydrous methylene chlorine (100 ml) kept under a nitrogen atmosphere and under stirring. When the addition is over the solvent was evaporated under vacuum and the viscous residue (6.6 g) was subjected to flash chromatography. By eluting with petroleum ether-ethyl ether 3:2, 1.25 g of the starting product was obtained. By further elution the ethyl 3α,7α-diacetoxy-22,23-methylene-5β-cholanate as a semisolid oil (3.2 g, yield calculated starting from the olefine 67%) was obtained.

IR (nujol) 1720 $cm^{-1}$.

NMR ($CDCl_3$): δ 0.60 (3H, s, C-18 Me), 0.93 (3H, s, C-19 Me), 1.2—1.4 (3H, m, —$CO_2CH_2\underline{CH_3}$), 2.02 (3H, s, C-7 $OCOCH_3$), 2.03 (3H, s, C-3 $OCOCH_3$), 3.9—4.3 (2H, m, —$CO_2\underline{CH_2}CH_3$), 4.3—4.7 (1H, m, C-3 CHOAC), 4.7—4.9 1H, m, C-7 CHOAC).

c) 3α,7α-Dihydroxy-22,23-methylene-5β-cholanic acid (Id)

A solution of NaOH 9.3 N (40 ml) and the ethyl ester obtained in b) (3.16 g, 6.1 mmol) in ethanol (60 ml) was refluxed under stirring. After 4 h the mixture was formed onto ice water (180 ml), extracted with ethyl ether, acidified with 10% hydrochloric acid and extracted with ethyl acetate (3 × 70 ml). The combined ethyl acetate extracts were washed with a saturated solution of sodium chloride and dried over $Na_2SO_4$ and the solvent evaporated under vacuum. There is obtained a solid residue (2.1 g, 85%).

m.p. 130—145°C.

IR (nujol) 3410, 1695 $cm^{-1}$.

NMR ($CD_3OD$) δ 0.65 (3H, s, C-18 Me), 0.93 (3H, s, C-19 Me), 3.1—3.5 (1H, m, C-3 CHOH), 3.6—3.8 (1H, m, C-7 CHOH).

The chemico-physical and pharmacological characteristics of 3α,7β-dihydroxy-5β-cholan-22,23-methylene-24-oic acid (Ic) have been studied in comparison with ursodeoxycholic acid.

Chemco-physical properties

Compound (Ic) showed values of solubility in water at 37°C (16 μM) and of $pK_a$ (4.7) very similar to those of ursodeoxycholic acid. The critic micellar concentration (CMC), determined by the method of azulene dissolution, proved to be however lower for Ic (5 mM) in comparison with the reference compound (8 mM), perhaps because of the lower hydrophobicity of the latter.

Pharmacological properties

The metabolism of (Ic) in rats has been studied in comparison to that of ursodeoxycholic acid, after endovenous administration of 50 mg/animal of both compounds, both as bolus and as slow infusion (1 mg/min). The bile was collected every 15 min for three hours. Only 70% of the compound (Ic) is conjugated with taurine in a single passage through the liver, while 30% is excreted as free acid; 95—97% of the reference compound is however excreted as the conjugate. Moreover (Ic) exhibits a clearly higher choleretic effect, along with a longer duration of action and an increase of the hourly secretion of the three biliary lipids, in a particularly selective way for phospholipids and biliary acids.

Therefore, thanks to their remarkable chloreretic effect and of the positive effect on the biliary secretion, above all in comparison with physiologic derivatives, the compounds of the invention are useful as choleretics, eupeptics, antidyspeptics, antidyslipidemics, in the treatment of calculosis and generally in all those pathologic conditions wherein a stimulus of the bile flow and a qualitative and quantitative modification of the biliary composition are necessary. An essential aspect of the present invention is therefore provided by pharmaceutical compositions containing as the active principle at least one of the compounds of formula I, in addition to the conventional pharmaceutical excipients. The compounds of the invention can be administered at doses ranging from 10 and 250 mg 2—3 times a day by the oral route, for instance in form of capsules, tablets, sugar-coated pills, monodose sachets or by local perfusin before surgical operations in form of solution of dispersable powders.

**Claims for the Contracting States: BE CH DE FR GB IT LI LU NL SE**

1. Biliary acids derivatives having formula I

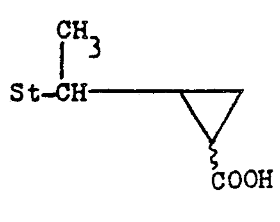

(I)

wherein St is a 17-etiocholanyl radical, having two or more hydroxyl groups in either the α and β configuration, some of which being optionally replaced by keto groups, and their pharmaceutically acceptable salts with alkaline or alkali-earth metals, or with organic bases and their stereoisomers.

2. A compound according to claim 1, which is 3α,7α,12α-trihydroxy-5β-cholan-22,23-methylene-cholan-24-oic acid (22,23-methylene-cholic acid) (Ia).

3. A compound according to claim 1, which is 3α,7β,12α-trihydroxy-5β-22,23-methylene-cholan-24-oic acid (Ib).

4. A compound according to claim 1, which is 3α,7β-dihydroxy-5β-22,23-methylene-cholan-24-oic acid (22,23-methylene-ursodeoxycholic acid) (Ic).

5. A compound according to claim 1, which is 3α,7α-dihydroxy-5β-22,23-methylene-cholan-24-oic acid (22,23-methylene-chenodeoxycholic acid) (Id).

6. A compound according to claim 1, which is 3α-hydroxy-7-keto-5β-22,23-methylene-cholan-24-oic acid (3α-hydroxy-7-keto-22,23-methylene-cholanic acid) (Ie).

7. Process for the preparation of compounds according to claims 1—6, characterized in that the esters of formula (II)

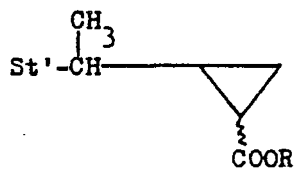

(II)

wherein St' represents a 17-etiocholanyl radical having two or more acyloxy groups in either the α and β configuration, some of which being optionally replaced by keto groups, and R is a $C_1$—$C_4$ alkyl radical, are subjected to hydrolysis.

8. Process according to claim 7, characterized in that the hydrolysis is carried out in a basic medium.

9. Pharmaceutical compositions characterized by containing as the active principle one or more compounds according to claims 1—6.

**Claims for the Contracting State: AT**

1. Process for the preparation of compounds of general formula (I)

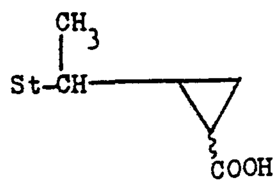

(I)

wherein St is a 17-etiocholanyl radical, having two or more hydroxyl groups in either the α and β configuration, some of which being optionally replaced by keto groups, characterized in that the esters of formula (II)

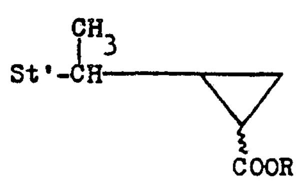

(II)

wherein St' represents a 17-etiocholanyl radical having two or more acyloxy groups in either the α and β configuration, some of which being optionally replaced by keto groups, and R is a $C_1$—$C_4$ alkyl radical, are subjected to hydrolysis.

2. Process according to claim 1, characterized in that the hydrolysis is carried out in a basic medium.

**Patentansprüche für die Vertragsstaaten: BE CH DE FR GB IT LI LU NL SE**

1. Gallensäurederivative der Formel I

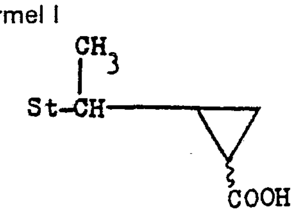

(I)

in welcher St einen 17-Etiocholanylrest mit zwei oder mehr Hydroxylgruppen in irgendeiner der α- und β-

Konfiguration, von welchen einige durch Ketogruppen ersetzt sein können, darstellt, und ihre pharmazeutisch annhembaren Salze mit Alkali- oder Erdalkalimetallen oder mit organisch Basen und ihre Stereoisomeren.

2. Eine Verbindung nach Anspruch 1, welche die 3α,7α,12α-Trihydroxy-5β-22,23-methylen-cholan-24-säure (22,23-Methylen-cholsäure) (Ia) ist.

3. Eine Verbindung nach Anspruch 1, welche die 3α,7β,12α-Trihydroxy-5β-22,23-methylen-cholan-24-säure (Ib) ist.

4. Eine Verbindung nach Anspruch 1, welche die 3α,7β-Dihdyroxy-5β-22,23-methylen-cholan-24-säure (22,23-Methylen-ursodesoxycholsäure) (Ic) ist.

5. Eine Verbindung nach Anspruch 1, welche die 3α,7α-Dihydroxy-5β-22,23-methylen-cholan-24-säure (22,23-Methylen-chenodesoxycholsäure (Id) ist.

6. Eine Verbindung nach Anspruch 1, welche die 3α-Hydroxy-7-keto-5β-22,23-methylen-cholan-säure (3α-Hydroxy-7-keto-22,23-methylen-cholansäure) (Ie) ist.

7. Verfahren zur Herstellung der Verbindungen nach den Ansprüchen 1 bis 6, dadurch gekennzeichnet, dass die Ester der Formel (II)

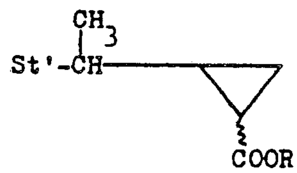

(II)

in welcher St′ einen 17-Etiocholanylrest mit zwei oder mehr Acyloxygruppen in der α- oder β-Konfiguration, von welchen einige durch Ketogruppen ersetzt sein können, darstellt und R eine $C_1$—$C_4$-Alkylrest ist, der Hydrolyse unterworfen werden.

8. Verfahren nach Anspruch 7, dadurch gekennzeichnet, dass die Hydrolyse in einem basischen Medium durchgeführt wird.

9. Pharmazeutische Zubereitungen, dadurch gekennzeichnet, dass sie als Wirkstoff eine oder mehrere Verbindungen nach Ansprüchen 1 bis 6 enthalten.

**Patentansprüche für den Vertragsstaat: AT**

1. Verfahren zur Herstellung der Verbindungen der allgemeinen Formel (I)

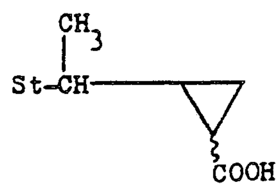

(I)

in welcher St einen 17-Etiocholanylrest mit zwei oder mehr Hydroxylgruppen in irgendeiner der α- und β-Konfiguration, von welchen einige durch Ketogruppen ersetzt sein können, darstellt, dadurch gekennzeichnet, dass die Ester der Formel (II)

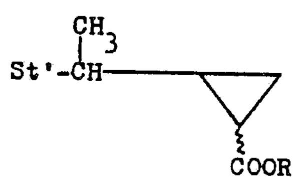

(II)

in welcher St′ einen 17-Etiocholanylrest mit zwei oder mehr Acyloxygruppen in der α- oder β-Konfiguration, von welchen einige durch Ketogruppen ersetzt sein können, darstellt und R eine $C_1$—$C_4$-Alkylrest ist, der Hydrolyse unterworfen werden.

2. Verfahren nach Anspruch 1, dadurch gekennzeichnet, dass die Hydrolyse in einem basischen Medium durchgeführt wird.

**Revendications pour les Etats contractants: BE CH DE FR GB IT LI LU NL SE**

1. Dérivés d'acides biliaires répondant à la formule I

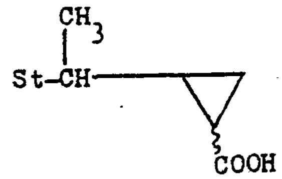

(I)

dans laquelle St représente un reste 17-étiocholanyle comportant deux ou plusieurs groupes hydroxyles dont quelquesuns sont remplacés cas échéant par des groupes cétoniqués, et leurs sels pharmaceutiquement acceptables avec des metaux alcalins ou alcalino-terreux ou avec des bases organiques, et leurs stéréoisomères.

2. Un composé selon la revendication 1, qui est l'acide 3α,7α,12α-trihydroxy-5β-22,23-méthylène-cholanique-(24) (acide 22,23-méthylène-cholique) (Ia).

3. Un composé selon la revendication 1, qui est l'acide 3α,7β,12α-trihydroxy-5β-22,23-méthylène-cholanique-(24) (Ib).

4. Un composé selon la revendication 1, qui est l'acide 3α,7β-dihydroxy-5β-22,23-méthylène-cholanique-(24) (acide 22,23-méthylène-ursodésoxycholique) (Ic).

5. Un composé selon la revendication 1, qui est l'acide 3α,7α-dihydroxy-5β-22,23-méthylène-cholanique-(24) (acide 22,23-méthylène-chénodésoxycholique) (Id).

6. Un composé selon la revendication 1, qui est l'acide 3α-hydroxy-7-céto-5β-22,23-méthylène-cholanique-(24) (acide 3α-hydroxy-7-céto-5β-22,23-méthylène-cholanique (Ie).

7. Procédé de préparation de composés selon les revendication 1 à 6, caractérisé en ce que les esters de formule (II)

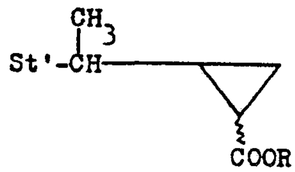

(II)

dans laquelle St' représente un radical 17-étiocholanyle comportant deux ou plusieurs groupes acyloxy en la configuration α ou β et dont quelques-uns sont remplacés cas échéant par des groupes cétoniques et R est un radical alkyle en $C_1$—$C_4$, sont soumis à l'hydrolyse.

8. Procédé selon la revendication 7, caractérisé en ce que l'hydrolyse est effectuée dans un milieu basique.

9. Compositions pharmaceutiques, caractérisées en ce qu'elles contiennent comme principe actif un ou plusieurs composés selon les revendications 1 à 6.

**Revendications pour l'Etat contractant: AT**

1. Procédé de préparation de composés de formule générale (I)

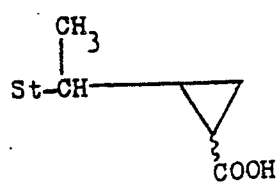

(I)

dans laquelle St représente un reste 17-étiocholanyle comportant deux ou plusieurs groupes hydroxyles en la configuration α ou β et dont quelques-uns sont remplacés cas échéant par des groupes cétoniques, caractérisé en ce que les esters de formule (II)

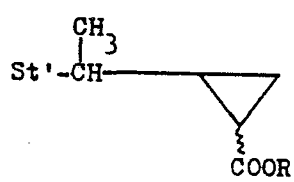

(II)

dans laquelle St' représente un radical 17-étiocholanyle comportant deux ou plusieurs groupes acyloxy en

la configuration α ou β et dont quelques-uns sont remplacés cas échéant par des groupes cétoniques et R est un radical alkyle en $C_1$—$C_4$, sont soumis à l'hydrolyse.

2. Procédé selon la revendication 1, caractérisé en ce que l'hydrolyse est effectuée dans un milieu basique.